# EUROPEAN PATENT APPLICATION

(11) **EP 3 603 418 A1**
(43) Date of publication of application: **05.02.2020**
(21) Application number: 18771074.4
(22) Date of filing: 22.03.2018
(51) Int. Cl.: A23L 29/269, A23L 29/20, A23L 29/30, A61K 9/16, A61K 9/20, A61K 47/36

(54) **METHOD FOR PRODUCING PREPARATION CONTAINING THICKENING POLYSACCHARIDE**

(30) Priority: 22.03.2017 JP 2017056515
(71) Applicant: San-Ei Gen F.F.I., INC., Toyonaka-shi, Osaka 561-8588 (JP)
(72) Inventor: SATO, Hiroyuki, Toyonaka-shi Osaka 561-8588 (JP)
(74) Representative: Vossius & Partner Patentanwälte Rechtsanwälte mbB
(86) International application number: PCT/JP2018/011544
(87) International publication number: WO 2018/174207

(57) **Abstract**

Provided are a method for producing a polysaccharide thickener-containing preparation in which formation of lumps is suppressed when the polysaccharide thickener-containing preparation is added to a liquid, and in which the inherent thickening function or gelling function of the polysaccharide thickener is exhibited satisfactorily; and the like.

The method for producing a polysaccharide thickener-containing preparation comprises granulating a polysaccharide thickener using one or more binder liquids, wherein a starch decomposition product having a DE of 1 or more and 50 or less, and at least one member selected from the group consisting of pullulan, guar gum, guar gum decomposition product, gum arabic, gum ghatti, soybean polysaccharides, pectin, and alginic acid and derivatives thereof are used as components of the one or more binder liquids.

## Description

### Technical Field

The present invention relates to a method for producing a polysaccharide thickener-containing preparation, and a method for improving the dispersibility of a polysaccharide thickener-containing preparation in a liquid.

### Background Art

Preparations containing a polysaccharide thickener, such as xanthan gum, have a function to thicken various compositions, including foods and drinks (thickening function); and a function to gelatinize various compositions (gelling function).

In order for polysaccharide thickeners to satisfactorily exhibit their thickening function or gelling function, they must be sufficiently swelled and hydrated. However, when a powdery or granular polysaccharide thickener is added to a liquid, such as water, only the surface of the powdery or granular polysaccharide thickener dissolves, and it remains in the form of a powder without swelling and hydration of the interior thereof; i.e., lumps are formed. Formed lumps are very difficult to completely dissolve. For example, even if the stirring speed is increased or the stirring time is increased, it is difficult to completely dissolve formed lumps.

For example, a technique focusing on the particle size of polysaccharide thickeners is known as a technique for suppressing formation of lumps (a lump-improving technique). As an example, Patent Literature 1 proposes a technique of granulating a xanthan gum powder having an average particle size of 80 µm or less.

However, fine powder-type xanthan gum having an average particle size of 80 µm or less has a problem in that the powder tends to scatter when handled. Further, a xanthan gum-containing preparation obtained based on this technique has unsatisfactory dispersibility in a liquid, and still has the problem such that lumps tend to form.

Another known lump-improving technique is a technique of granulating a polysaccharide thickener using a binder liquid. For example, Patent Literature 2 proposes a technique of spraying a binder liquid containing 18 to 35 mass% dextrin onto a powder containing a polysaccharide thickener and dextrin for granulation. Patent Literature 3 proposes a technique of spraying a solution containing a guar gum enzymatic decomposition product onto a xanthan gum powder, and then performing fluidized bed drying. Patent Literature 4 discloses, as Comparative Example 4, an example in which a xanthan gum powder was granulated using a binder liquid containing gum arabic. Patent Literature 5 discloses a preparation obtained by granulating a xanthan gum powder using a binder liquid containing gum arabic; and then forming a mixture of the obtained granulated product, a disintegrator, and an excipient into a tablet.

However, the dispersibility improvement effect of polysaccharide thickeners obtained by the techniques disclosed in Patent Literature 2 to Patent Literature 5 is not sufficient, and the Patent Literature nowhere discloses focusing on the specific combination of the present invention.

### Citation List

### Patent Literature

PTL 1: JP2006-304605A
PTL 2: JP2011-120538A
PTL 3: JP2007-110983A
PTL 4: JP4672596B
PTL 5: JP2015-136315A

### Summary of Invention

### Technical Problem

An object of the present invention is to provide a method for producing a polysaccharide thickener-containing preparation in which dispersibility in a liquid is improved, and formation of lumps is suppressed.

Lumps are formed due to the rapid swelling and hydration (dissolution) rates of polysaccharide thickeners. Thus, conventional techniques aimed at suppressing formation of lumps have a problem in that they slow the dissolution rate of polysaccharide thickeners, resulting in a decrease in the inherent thickening function or gelling function of polysaccharide thickeners.

The present invention has been accomplished in view of this problem, as well. An object of the present invention is to provide a method for producing a polysaccharide thickener-containing preparation in which formation of lumps is suppressed, and the polysaccharide thickener satisfactorily exhibits its inherent thickening function or gelling function.

### Solution to Problem

The present inventors conducted extensive research in view of the above prior art, and found that a polysaccharide thickener-containing preparation in which dispersibility in a liquid is improved and formation of lumps is suppressed can be provided by granulating a polysaccharide thickener using a starch decomposition product having a DE of 1 or more and 50 or less, and at least one member selected from the group consisting of pullulan, guar gum, guar gum decomposition product, gum arabic, gum ghatti, soybean polysaccharides, pectin, and alginic acid and derivatives thereof as components of one or more binder liquids. The present inventors also found that a polysaccharide thickener-containing preparation produced using the present invention exhibits an excellent thickening function or gelling function. As a result of further research, the present invention has been accomplished.

The present invention relates to the following methods for producing a polysaccharide thickener-containing preparation, and methods for improving the dispersibility of a polysaccharide thickener-containing preparation in a liquid.

### (1) Method for producing polysaccharide thickener-containing preparation

Item 1-1. A method for producing a polysaccharide thickener-containing preparation, comprising the step of granulating a polysaccharide thickener using one or more binder liquids, wherein a starch decomposition product having a DE of 1 or more and 50 or less, and at least one member selected from the group consisting of pullulan, guar gum, guar gum decomposition product, gum arabic, gum ghatti, soybean polysaccharides, pectin, and alginic acid and derivatives thereof are used as components of the one or more binder liquids.
Item 1-2. The method for producing a polysaccharide thickener-containing preparation according to Item 1-1, wherein a binder liquid comprising a starch decomposition product having a DE of 1 or more and 50 or less, and at least one member selected from the group consisting of pullulan, guar gum, guar gum decomposition product, gum arabic, gum ghatti, soybean polysaccharides, pectin, and alginic acid and derivatives thereof is used in the granulation step.
Item 1-3. The method for producing a polysaccharide thickener-containing preparation according to Item 1-1 or 1-2, wherein the polysaccharide thickener comprises at least one member selected from the group consisting of xanthan gum, carrageenan, galactomannan, welan gum, pectin, gellan gum, alginic acid and derivatives thereof, and starches.
Item 1-4. The method for producing a polysaccharide thickener-containing preparation according to any one of Items 1-1 to 1-3, wherein the proportion of the starch decomposition product having a DE of 1 or more and 50 or less relative to the at least one member selected from the group consisting of pullulan, guar gum, guar gum decomposition product, gum arabic, gum ghatti, soybean polysaccharides, pectin, and alginic acid and derivatives thereof, used as the components of the one or more binder liquids, is such that the amount of the starch decomposition product having a DE of 1 or more and 50 or less is 0.03 to 40 parts by mass, per part by mass of the at least one member selected from the group consisting of pullulan, guar gum, guar gum decomposition product, gum arabic, gum ghatti, soybean polysaccharides, pectin, and alginic acid and derivatives thereof.

### (2) Method for improving dispersibility of polysaccharide thickener-containing preparation in liquid

Item 2-1. A method for improving the dispersibility of a polysaccharide thickener-containing preparation in a liquid, comprising the step of granulating a polysaccharide thickener using one or more binder liquids, wherein a starch decomposition product having a DE of 1 or more and 50 or less, and at least one member selected from the group consisting of pullulan, guar gum, guar gum decomposition product, gum arabic, gum ghatti, soybean polysaccharides, pectin, and alginic acid and derivatives thereof are used as components of the one or more binder liquids.
Item 2-2. The method according to Item 2-1, wherein a binder liquid comprising a starch decomposition product having a DE of 1 or more and 50 or less, and at least one member selected from the group consisting of pullulan, guar gum, guar gum decomposition product, gum arabic, gum ghatti, soybean polysaccharides, pectin, and alginic acid and derivatives thereof is used in the granulation step.
Item 2-3. The method according to Item 2-1 or 2-2, wherein the polysaccharide thickener comprises at least one member selected from the group consisting of xanthan gum, carrageenan, galactomannan, welan gum, pectin, gellan gum, alginic acid and derivatives thereof, and starches.
Item 2-4. The method according to any one of Items 2-1 to 2-3, wherein the proportion of the starch decomposition product having a DE of 1 or more and 50 or less relative to the at least one member selected from the group consisting of pullulan, guar gum, guar gum decomposition product, gum arabic, gum ghatti, soybean polysaccharides, pectin, and alginic acid and derivatives thereof, used as the components of the one or more binder liquids, is such that the amount of the starch decomposition product having a DE of 1 or more and 50 or less is 0.03 to 40 parts by mass, per part by mass of the at least one member selected from the group consisting of pullulan, guar gum, guar gum decomposition product, gum arabic, gum ghatti, soybean polysaccharides, pectin, and alginic acid and derivatives thereof.
Item 2-5. The method according to any one of Items 2-1 to 2-4, wherein the polysaccharide thickener-containing preparation further has a property of increasing the viscosity of a liquid.

### Advantageous Effects of Invention

The present invention provides a polysaccharide thickener-containing preparation in which dispersibility in a liquid is improved, and formation of lumps is suppressed. The present invention enables suppression of formation of lumps, and thus provides a polysaccharide thickener-containing preparation that can exhibit an excellent thickening function or gelling function for a liquid, such as water or milk.

### Brief Description of Drawings

Fig. 1 is a photograph that shows the results of a dispersibility test of the polysaccharide thickener-containing preparation of Example 1-1 in Experiment Example 1.
Fig. 2 is a photograph that shows the results of a dispersibility test of the polysaccharide thickener-containing preparation of Comparative Example 1 in Experiment Example 1.
Fig. 3 is a set of photographs that show the results of a dispersibility test of the polysaccharide thickener-containing preparations of Example 6-2 and Comparative Example 6-2 in Experiment Example 6.
Fig. 4 is a set of photographs that show the results of a dispersibility test of the polysaccharide thickener-containing preparations of Examples 7-1 to 7-4, and Comparative Examples 7-1 and 7-2 in Experiment Example 7.
Fig. 5 is a set of photographs that show the results of a dispersibility test of the polysaccharide thickener-containing preparations of Examples 8-1 to 8-6 in Experiment Example 8.
Fig. 6 is a set of photographs that show the results of a dispersibility test of the polysaccharide thickener-containing preparations of Examples 9-1 and 9-2 in Experiment Example 9.

### Description of Embodiments

The present invention relates to a method for producing a polysaccharide thickener-containing preparation, comprising the step of granulating a polysaccharide thickener using one or more binder liquids, wherein a starch decomposition product having a DE of 1 or more and 50 or less, and at least one member selected from the group consisting of pullulan, guar gum, guar gum decomposition product, gum arabic, gum ghatti, soybean polysaccharides, pectin, and alginic acid and derivatives thereof are used as components of the binder liquid(s).

The polysaccharide thickener used in the present invention is not particularly limited. In the present invention, an edible polysaccharide thickener that is allowed to be used in a food or drink, or a pharmaceutical product for oral administration, is preferably used.

Examples of polysaccharide thickeners include xanthan gum, carrageenan (e.g., κ(kappa)-carrageenan, ι(iota)-carrageenan, and λ(lambda)-carrageenan), galactomannan (e.g., locust bean gum, tara gum, and guar gum), alginic acid and derivatives thereof (e.g., alginic acid, alginic acid salts (e.g., sodium alginate, potassium alginate, calcium alginate, and ammonium alginate), and alginic acid esters (e.g., propylene glycol alginate)), pectin (e.g., HM (high-methoxyl) pectin and LM (low-methoxyl) pectin), gellan gum (e.g., deacylated gellan gum and native gellan gum), gum arabic, pullulan, agar, gelatin, tamarind seed gum, glucomannan, welan gum, psyllium seed gum, karaya gum, curdlan, starches (e.g., unmodified starch (raw starch), pregelatinized starch, modified/chemically modified starch, and physically modified starch), furcellaran, fermented cellulose, crystalline cellulose, soybean polysaccharides, succinoglycan, and the like. These polysaccharide thickeners may be used singly, or in a combination of two or more.

Among the above polysaccharide thickeners, xanthan gum, carrageenan, galactomannan, welan gum, pectin, gellan gum, alginic acid and derivatives thereof, and starches, particularly xanthan gum and starches, have high swelling and hydration rates; therefore, it is extremely difficult to suppress formation of lumps in these polysaccharide thickeners. However, according to the present invention, formation of lumps upon addition of a preparation to a liquid can be significantly suppressed even if the preparation contains such a polysaccharide thickener, and a polysaccharide thickener-containing preparation having excellent dispersibility can be provided. From this viewpoint, the present invention can be suitably used for production of a preparation containing at least one member selected from the group consisting of xanthan gum, carrageenan, galactomannan, welan gum, pectin, gellan gum, alginic acid and derivatives thereof, and starches as a polysaccharide thickener; and can be more suitably used for production of a preparation containing xanthan gum and/or starch as a polysaccharide thickener.

In the present invention, it is preferable to use a powdery or granular polysaccharide thickener as a granulation raw material. The granulation raw material may further optionally comprise an excipient etc., in addition to a polysaccharide thickener. It is only necessary that the granulation raw material comprises a polysaccharide thickener. That is, the granulation raw material may consist of a polysaccharide thickener; or may further optionally comprise an excipient etc. with a polysaccharide thickener.

The method for producing a polysaccharide thickener-containing preparation according to the present invention comprises the step of granulating a polysaccharide thickener using one or more binder liquids. However, the granulation method is not particularly limited. Examples of the granulation method include fluidized bed granulation, stirring granulation, extrusion granulation, rotating granulation, and the like. The granulation is preferably fluidized bed granulation, or stirring granulation.

Granulation by fluidized bed granulation is not particularly limited, and may be performed according to a usual method. For example, one embodiment of granulation by fluidized bed granulation is described below.

A raw material for granulation (granulation raw material) comprising a powdery or granular polysaccharide thickener is placed in a granulator, and hot air is fed from below the machine to allow the granulation raw material to flow. One or more binder liquids are sprayed onto the fluidized bed of the granulation raw material to adhere the binder liquid(s) to the surface of the granulation raw material, thereby forming aggregated granules of the granulation raw material. The aggregated granules are dried to prepare a granulated product.

Granulation by stirring granulation is also not particularly limited, and may be performed according to a usual method. For example, one embodiment of granulation by stirring granulation is described below.

A granulation raw material comprising a powdery or granular polysaccharide thickener is placed in a granulator; and one or more binder liquids are sprayed or directly added, followed by rapid stirring. Aggregated granules of the granulation raw material are formed by the stirring, and dried to prepare a granulated product.

Granulation by other methods, such as extrusion granulation and rotating granulation, is also not particularly limited; and may be performed according to a usual method.

In the present invention, a powdery or granular granulation raw material is preferable as a granulation raw material.

In the present invention, a starch decomposition product having a DE of 1 or more and 50 or less, and at least one member selected from the group consisting of pullulan, guar gum, guar gum decomposition product, gum arabic, gum ghatti, soybean polysaccharides, pectin, and alginic acid and derivatives thereof are used as components of one or more binder liquids used in the granulation step. In the present invention, a starch decomposition product having a DE of 1 or more and 50 or less, and at least one member selected from the group consisting of pullulan, guar gum, guar gum decomposition product, gum arabic, gum ghatti, soybean polysaccharides, pectin, and alginic acid and derivatives thereof used as components of a binder liquid may be contained in one binder liquid, or the components may be individually contained in separate binder liquids. The components of the binder liquid(s) are, for example, more preferably a starch decomposition product having a DE of 1 or more and 50 or less, and at least one member selected from the group consisting of pullulan, guar gum, gum arabic, and soybean polysaccharides. However, the present invention is not limited thereto.

The pullulan used in the present invention is a known α-glucan produced by *Aureobasidium pullulans* (filamentous fungus), which is a black yeast. The pullulan is a linear polysaccharide with a structure in which maltotriose is regularly linked by α-1,6-glucosidic bonds as a repeating units.

The guar gum used in the present invention is a known polysaccharide obtained from the endosperm of seeds of leguminous plant guar cultivated mainly in South Asia. The guar gum is known to have a molecular structure in which D-galactose as a side chain is α-1,6-linked to the main-chain backbone of β-1,4-D-mannan, and the ratio of mannose and galactose in the guar gum is about 2:1. The guar gum is commercially available. Examples of commercially available products thereof include VIS TOP (registered trademark) D-20, produced by San-Ei Gen F.F.I., Inc., and the like.

The guar gum decomposition product used in the present invention is known and obtained by hydrolyzing guar gum to reduce the molecular weight thereof. The hydrolysis method is not limited, and is, for examples, an enzymatic decomposition method, an acid decomposition method, or the like; and preferably an enzymatic decomposition method. The guar gum decomposition product may be one whose molecular weight is adjusted by performing a treatment with a membrane, or using a column for molecular weight fractionation in combination with such a method. There is no limitation on the enzyme used for an enzymatic decomposition method, as long as the enzyme is capable of hydrolyzing a mannose straight chain. Such enzymes are known, and preferable examples thereof include β-*mannanase.*

The gum arabic used in the present invention is a known polysaccharide obtained from sap of a plant of the genus *Acacia* of the Leguminosae family (e.g., *Acacia senegal* or *Acacia seyal*). Although the molecular structure of the gum arabic is not fully clarified, the gum arabic is known to contain galactose, arabinose, rhamnose, and glucuronic acid as constituent sugars. The gum arabic is commercially available. Examples of commercially available products thereof include Gum Arabic SD, produced by San-Ei Gen F.F.I., Inc., and the like.

In the present invention, modified gum arabic obtained by modifying the gum arabic described above is also usable as the gum arabic. Examples of the method for modifying gum arabic include a method in which a heat treatment is performed at 90 to 180°C for 15 minutes to 72 hours, a method in which metal salts are removed from or reduced in gum arabic, and the like. Examples of the method in which metal salts are removed from or reduced in gum arabic include an ion-exchange treatment in an organic solvent; and a desalting treatment using, for example, an electrodialysis membrane or an ion-exchange resin. Such modified gum arabic can be produced according to a method described in, for example, JP2006-522202A or JP2005-179417A.

The gum ghatti used in the present invention is a known, naturally derived plant gum substance that contains, as a major ingredient, polysaccharide obtained by drying secretory fluid from the trunk of *Anogeissus latifolia* Wall. of the Combretaceae family. The gum ghatti is commercially available. Examples of commercially available products thereof include Gum Ghatti SD, produced by San-Ei Gen F.F.I., Inc., and the like.

The soybean polysaccharides used in the present invention are known, and are not particularly limited as long as they are polysaccharides extracted from soybeans. For example, soybean polysaccharides extracted from water-soluble dietary fiber (soy pulp) produced in the process of producing isolated soybean protein are usable. The soybean polysaccharides can be produced by a method described in, for example, JPH05-032701A. The soybean polysaccharides presumably have a molecular structure in which they are composed of sugars such as galactose, arabinose, galacturonic acid, rhamnose, xylose, fucose, and glucose; and in which galactan and arabinan are bound to a rhamnogalacturonic acid chain. The soybean polysaccharides are commercially available. Examples of commercially available products thereof include SM-700 and SM-1200, produced by San-Ei Gen F.F.I., Inc., and the like.

The pectin used in the present invention is a known polysaccharide mainly composed of galacturonic acid and its methyl ester. Pectin is roughly classified into HM (high-methoxyl) pectin and LM (low-methoxyl) pectin, depending on the proportion of galacturonic acid in the ester form (DE = degree of esterification). In general, pectin having a DE of 50% or more is called "HM pectin," and pectin having a DE of less than 50% is called "LM pectin." HM pectin and LM pectin are both usable as the pectin used in the present invention. The pectin is commercially available. Examples of commercially available products thereof include VIS TOP (registered trademark) D-402, produced by San-Ei Gen F.F.I., Inc., and the like.

The alginic acid and derivatives thereof used in the present invention are known linear polysaccharides that are obtained from brown algae and are composed of uronic acids, which have COOH at the C-6 position. The alginic acid and derivatives thereof are explained as described above. The alginic acid and derivatives thereof are commercially available. Examples of commercially available products thereof include SAN SUPPORT (registered trademark) P-90, produced by San-Ei Gen F.F.I., Inc., and the like.

The total content of the at least one member selected from the group consisting of pullulan, guar gum, guar gum decomposition product, gum arabic, gum ghatti, soybean polysaccharides, pectin, and alginic acid and derivatives thereof in a binder liquid used in the present invention is not particularly limited; and is, for example, 0.01 to 20 mass%, preferably 0.02 to 18 mass%, more preferably 0.03 to 15 mass%, further preferably 0.05 to 12 mass%, further more preferably 0.08 to 10 mass%, and particularly preferably 0.1 to 10 mass% in the binder liquid. Moreover, the total content of the at least one member selected from the group consisting of pullulan, guar gum, guar gum decomposition product, gum arabic, gum ghatti, soybean polysaccharides, pectin, and alginic acid and derivatives thereof in a binder liquids used in the present invention is not particularly limited; and may be, for example, 0.1 to 20 mass%, 0.2 to 18 mass%, 0.3 to 15 mass%, 0.5 to 12 mass%, 0.8 to 10 mass%, 1 to 10 mass%, or the like, in the binder liquid.

The starch decomposition product having a DE of 1 or more and 50 or less used in the present invention is a known intermediate obtained by hydrolyzing a pasty liquid obtained by heating starch with water, using an acid or an enzyme. The degree of decomposition of the starch decomposition product can be indicated by a DE (dextrose equivalent) value, which is used as an indicator of decomposition in this field. The starch decomposition product used in the present invention generally has a DE of 1 or more, preferably 2 or more, and more preferably 3 or more. The starch decomposition product used in the present invention also generally has a DE of 50 or less, preferably 40 or less, more preferably less than 40, further preferably 35 or less, further more preferably 30 or less, and particularly preferably 25 or less. From this point of view, the starch decomposition product used in the present invention more preferably has, for example, a DE of 2 or more and 40 or less, further preferably 2 or more and 35 or less, further more preferably 2 or more and 30 or less, and particularly preferably 2 or more and 25 or less. The use of a starch decomposition product having a DE in this range makes it possible to produce a polysaccharide thickener-containing preparation in which dispersibility in a liquid is improved, and formation of lumps is suppressed. Starch decomposition products having a DE of 1 or more and 50 or less may be used singly, or in a combination of two or more.

The total content of the starch decomposition product having a DE of 1 or more and 50 or less in a binder liquid used in the present invention is not particularly limited; and is, for example, 0.1 to 20 mass%, preferably 0.2 to 18 mass%, more preferably 0.3 to 15 mass%, further preferably 0.5 to 12 mass%, further more preferably 0.8 to 10 mass%, and particularly preferably 1 to 10 mass%, in the binder liquid.

The one or more binder liquids can be prepared by dissolving in water at least one member selected from the group consisting of pullulan, guar gum, guar gum decomposition product, gum arabic, gum ghatti, soybean polysaccharides, pectin, alginic acid and derivatives thereof, and starch decomposition products having a DE of 1 or more and 50 or less. Thus, in the present invention, the one or more binder liquids containing at least one member selected from the group consisting of pullulan, guar gum, guar gum decomposition product, gum arabic, gum ghatti, soybean polysaccharides, pectin, alginic acid and derivatives thereof, and starch decomposition products having a DE of 1 or more and 50 or less preferably contain water. Since pullulan, guar gum, guar gum decomposition product, gum arabic, gum ghatti, soybean polysaccharides, pectin, alginic acid and derivatives thereof, and starch decomposition products having a DE of 1 or more and 50 or less are all soluble in water at a temperature around room temperature (e.g., 20°C), a heating process is unnecessary in preparing the binder liquid(s). However, liquid(s) containing at least one member selected from the group consisting of pullulan, guar gum, guar gum decomposition product, gum arabic, gum ghatti, soybean polysaccharides, pectin, alginic acid and derivatives thereof, and starch decomposition products having a DE of 1 or more and 50 or less may be heated, if necessary.

The binder liquid(s) used in the present invention may contain an alcohol having 3 or fewer carbon atoms (e.g., methanol, ethanol, or glycerin), a metal salt, an organic acid, or the like.

In the step of granulating a polysaccharide thickener using one or more binder liquids in the present invention, a starch decomposition product having a DE of 1 or more and 50 or less, and at least one member selected from the group consisting of pullulan, guar gum, guar gum decomposition product, gum arabic, gum ghatti, soybean polysaccharides, pectin, and alginic acid and derivatives thereof are used as components of the binder liquid(s). The method of using the binder liquid(s) is not particularly limited.

For example, in the fluidized bed granulation or stirring granulation described above, the following methods, for example, can be used:
(i) a method in which a binder liquid containing a starch decomposition product having a DE of 1 or more and 50 or less, and at least one member selected from the group consisting of pullulan, guar gum, guar gum decomposition product, gum arabic, gum ghatti, soybean polysaccharides, pectin, and alginic acid and derivatives thereof is sprayed onto a granulation raw material comprising a powdery or granular polysaccharide thickener;
(ii) a method in which a binder liquid containing at least one member selected from the group consisting of pullulan, guar gum, guar gum decomposition product, gum arabic, gum ghatti, soybean polysaccharides, pectin, and alginic acid and derivatives thereof is sprayed onto a granulation raw material comprising a powdery or granular polysaccharide thickener, and a binder liquid containing a starch decomposition product having a DE of 1 or more and 50 or less is then sprayed; or
(iii) a method in which a binder liquid containing a starch decomposition product having a DE of 1 or more and 50 or less is sprayed onto a granulation raw material comprising a powdery or granular polysaccharide thickener, and a binder liquid containing at least one member selected from the group consisting of pullulan, guar gum, guar gum decomposition product, gum arabic, gum ghatti, soybean polysaccharides, pectin, and alginic acid and derivatives thereof is then sprayed.

In the case of the stirring granulation, for example, the following methods can be used in addition to the above binder liquid spraying methods:
(iv) a method in which a binder liquid containing a starch decomposition product having a DE of 1 or more and 50 or less, and at least one member selected from the group consisting of pullulan, guar gum, guar gum decomposition product, gum arabic, gum ghatti, soybean polysaccharides, pectin, and alginic acid and derivatives thereof is added to a granulation raw material comprising a polysaccharide thickener;
(v) a method in which a binder liquid containing at least one member selected from the group consisting of pullulan, guar gum, guar gum decomposition product, gum arabic, gum ghatti, soybean polysaccharides, pectin, and alginic acid and derivatives thereof is added to a granulation raw material comprising a polysaccharide thickener, and a binder liquid containing a starch decomposition product having a DE of 1 or more and 50 or less is then added; or
(vi) a method in which a binder liquid containing a starch decomposition product having a DE of 1 or more and 50 or less is added to a granulation raw material comprising a polysaccharide thickener; and a binder liquid containing at least one member selected from the group consisting of pullulan, guar gum, guar gum decomposition product, gum arabic, gum ghatti, soybean polysaccharides, pectin, and alginic acid and derivatives thereof is then added.

From the viewpoint of further improving the dispersibility of a polysaccharide thickener-containing preparation in a liquid, it is preferable to use one or more binder liquids containing a starch decomposition product having a DE of 1 or more and 50 or less, and at least one member selected from the group consisting of pullulan, guar gum, guar gum decomposition product, gum arabic, gum ghatti, soybean polysaccharides, pectin, and alginic acid and derivatives thereof in the granulation step (for example, methods (i) and (iv) above).

The amount of binder liquid(s) relative to polysaccharide thickener during granulation is not particularly limited. The amount of binder liquid(s) is preferably 0.1 to 4 parts by mass, more preferably 0.12 to 3.5 parts by mass, and even more preferably 0.15 to 3 parts by mass, per part by mass of the polysaccharide thickener in the granulation raw material. The amount of binder liquid(s) during granulation is not particularly limited; and is, for example, preferably 0.1 to 4 parts by mass, more preferably 0.12 to 3.5 parts by mass, and even more preferably 0.15 to 3 parts by mass, per part by mass of the granulation raw material.

As described above, in the step of granulating a polysaccharide thickener using one or more binder liquids in the present invention, the use of a starch decomposition product having a DE of 1 or more and 50 or less, and at least one member selected from the group consisting of pullulan, guar gum, guar gum decomposition product, gum arabic, gum ghatti, soybean polysaccharides, pectin, and alginic acid and derivatives thereof as components of the binder liquid(s) makes it possible to produce a polysaccharide thickener-containing preparation that has excellent dispersibility in a liquid, and thus is excellent in exhibiting functions (thickening function and/or gelling function) inherent to the polysaccharide thickener (effectively exhibits functions inherent to the polysaccharide thickener).

The proportions of the starch decomposition product having a DE of 1 or more and 50 or less, and at least one member selected from the group consisting of pullulan, guar gum, guar gum decomposition product, gum arabic, gum ghatti, soybean polysaccharides, pectin, and alginic acid and derivatives thereof used as components of the binder liquid(s) are not particularly limited. For example, the total amount (proportion) of starch decomposition product having a DE of 1 or more and 50 or less is preferably 0.03 to 40 parts by mass, more preferably 0.05 to 35 parts by mass, further preferably 0.1 to 32 parts by mass, further more preferably 0.12 to 30 parts by mass, particularly preferably 0.15 to 28 parts by mass, and more particularly preferably 0.15 to 25 parts by mass, per part by mass of at least one member selected from the group consisting of pullulan, guar gum, guar gum decomposition product, gum arabic, gum ghatti, soybean polysaccharides, pectin, and alginic acid and derivatives thereof (in total) in the total binder liquid amount. Although not limited in the present invention, for example, the total amount (proportion) of starch decomposition product having a DE of 1 or more and 50 or less may be, for example, 0.05 to 20 parts by mass, 0.1 to 15 parts by mass, 0.12 to 10 parts by mass, 0.15 to 8 parts by mass, 0.15 to 6 parts by mass, or the like, per part by mass of at least one member selected from the group consisting of pullulan, guar gum, guar gum decomposition product, gum arabic, gum ghatti, soybean polysaccharides, pectin, and alginic acid and derivatives thereof (in total) in the total binder liquid amount.

In the present invention, the use of a starch decomposition product having a DE of 1 or more and 50 or less, and at least one member selected from the group consisting of pullulan, guar gum, guar gum decomposition product, gum arabic, gum ghatti, soybean polysaccharides, pectin, and alginic acid and derivatives thereof in combination in the proportions described above as components of the binder liquid(s) enables a further improvement in the dispersibility of the obtained polysaccharide thickener-containing preparation, and thus allows functions inherent to the polysaccharide thickener to be sufficiently exhibited.

In the present invention, it is preferable that the amounts of starch decomposition product having a DE of 1 or more and 50 or less, and at least one member selected from the group consisting of pullulan, guar gum, guar gum decomposition product, gum arabic, gum ghatti, soybean polysaccharides, pectin, and alginic acid and derivatives thereof in the binder liquid(s); as well as the amount of binder liquid(s) relative to the polysaccharide thickener in the granulation raw material, are suitably adjusted so that the total content of the starch decomposition product having a DE of 1 or more and 50 or less, and at least one member selected from the group consisting of pullulan, guar gum, guar gum decomposition product, gum arabic, gum ghatti, soybean polysaccharides, pectin, and alginic acid and derivatives thereof derived from the binder liquid(s) in the polysaccharide thickener-containing preparation is, for example, 0.3 to 15 mass%, preferably 0.3 to 12 mass%, more preferably 0.4 to 12 mass%, further preferably 0.4 to 10 mass%, further more preferably 0.5 to 10 mass%, and particularly preferably 0.5 to 8 mass%.

The total content of the polysaccharide thickener derived from the granulation raw material in the polysaccharide thickener-containing preparation obtained by the production method of the present invention is not particularly limited; and is, for example, 5 to 90 mass%, preferably 10 to 85 mass%, more preferably 10 to 80 mass%, further preferably 15 to 80 mass%, further more preferably 18 to 80 mass%, further particularly preferably 18 to 75 mass%, further more particularly preferably 20 to 75 mass%, and further even more particularly preferably 25 to 75 mass%. The phrase "derived from the granulation raw material" in the present invention means, in other words, "not derived from the binder liquid(s) used in the production." In addition, the content of the "polysaccharide thickener derived from the granulation raw material in the polysaccharide thickener-containing preparation" according to the present invention is a value that does not contain dextrin.

When the polysaccharide thickener derived from the granulation raw material comprises at least one member selected from the group consisting of xanthan gum, carrageenan, galactomannan, welan gum, pectin, gellan gum, alginic acid and derivatives thereof, and starches, the total content of the at least one polysaccharide thickener selected from the group consisting of xanthan gum, carrageenan, galactomannan, welan gum, pectin, gellan gum, alginic acid and derivatives thereof, and starches derived from the granulation raw material in the polysaccharide thickener-containing preparation obtained by the production method of the present invention is, for example, preferably 5 to 90 mass%, more preferably 10 to 85 mass%, further preferably 10 to 80 mass%, further more preferably 15 to 80 mass%, particularly preferably 18 to 80 mass%, more particularly preferably 18 to 75 mass%, even more particularly preferably 20 to 75 mass%, and still even more particularly preferably 25 to 75 mass%. However, the present invention is not limited thereto.

Among polysaccharide thickeners, xanthan gum and starch have an excellent viscosity-increasing property in a liquid, such as water; and are useful as a thickening agent etc. However, these polysaccharide thickeners have a problem in that lumps are more likely to be formed. However, according to the production method of the present invention, a polysaccharide thickener-containing preparation in which formation of lumps of xanthan gum and/or starch is suppressed and an excellent thickening function is exhibited can be provided, even when the total content of these polysaccharide thickeners (i.e., the total content of xanthan gum and/or starch) derived from the granulation raw material in the polysaccharide thickener-containing preparation is as high as 5 mass% or more, 8 mass% or more, 10 mass% or more, 12 mass% or more, 15 mass% or more, 18 mass% or more, 20 mass% or more, or 25 mass% or more.

The upper limit of the total content of these polysaccharide thickeners (the total content of xanthan gum and/or starch) derived from the granulation raw material in the polysaccharide thickener-containing preparation is not particularly limited; and is, for example, 90 mass% or less, 88 mass% or less, 85 mass% or less, 82 mass% or less, 80 mass% or less, 78 mass% or less, or 75 mass% or less.

From these points of view, when the polysaccharide thickener-containing preparation obtained by the production method of the present invention contains xanthan gum and/or starch derived from the granulation raw material, the total content of these polysaccharide thickeners (xanthan gum and/or starch) derived from the granulation raw material in the polysaccharide thickener-containing preparation is, for example, preferably 5 to 90 mass%, more preferably 8 to 88 mass%, further preferably 10 to 85 mass%, further more preferably 12 to 82 mass%, particularly preferably 15 to 80 mass%, further particularly preferably 18 to 78 mass%, further more particularly preferably 20 to 78 mass%, further even more particularly preferably 20 to 75 mass%, and further still even more particularly preferably 25 to 75 mass%. However, the present invention is not limited thereto.

Moreover, the polysaccharide thickener-containing preparation of the present invention preferably contains an excipient. The timing of adding an excipient in the method for producing a polysaccharide thickener-containing preparation according to the present invention is not particularly limited. For example, the polysaccharide thickener-containing preparation may be produced by incorporating an excipient as a component of the granulation raw material when granulating a polysaccharide thickener, or mixing an excipient with a polysaccharide thickener after granulation.

Examples of excipients include starch decomposition products (e.g., dextrin and powdered starch syrup), saccharides (e.g., monosaccharides such as glucose and fructose; disaccharides such as sucrose, lactose, maltose, and trehalose; oligosaccharides such as cellooligosaccharides, maltooligosaccharides, and fructooligosaccharides; sugar alcohols such as xylitol, sorbitol, lactitol, and maltitol; and hydrogenated starch hydrolysates), dietary fibers (e.g., indigestible dextrin, polydextrose, and guar gum enzymatic decomposition product), and the like. These may be used singly, or in a combination of two or more. When an excipient is used, the excipient content in the polysaccharide thickener-containing preparation is not particularly limited; and is, for example, 9 to 90 mass% or 10 to 90 mass%. The excipient content is preferably 15 to 85 mass%, more preferably 18 to 80 mass%, further preferably 20 to 80 mass%, particularly preferably 20 to 75 mass%, more particularly preferably 25 to 78 mass%, and further preferably 25 to 70 mass%.

The granulation raw material in the present invention may or may not comprise at least one member selected from the group consisting of pullulan, guar gum, guar gum decomposition product, gum arabic, gum ghatti, soybean polysaccharides, pectin, alginic acid and derivatives thereof, and starch decomposition products having a DE of 1 or more and 50 or less. However, the present invention is not limited thereto. When the granulation raw material comprises at least one member selected from the group consisting of pullulan, guar gum, guar gum decomposition product, gum arabic, gum ghatti, soybean polysaccharides, pectin, and alginic acid and derivatives thereof, the total content of the at least one member selected from the group consisting of pullulan, guar gum, guar gum decomposition product, gum arabic, gum ghatti, soybean polysaccharides, pectin, and alginic acid and derivatives thereof derived from the granulation raw material of the polysaccharide thickener-containing preparation obtained by the production method of the present invention is, for example, 0.1 to 80 mass%, preferably 0.1 to 70 mass%, more preferably 0.1 to 65 mass%, further preferably 0.2 to 65 mass%, further more preferably 0.3 to 60 mass%, particularly preferably 0.3 to 55 mass%. When the granulation raw material comprises a starch decomposition product having a DE of 1 or more and 50 or less, the content of the starch decomposition product having a DE of 1 or more and 50 or less derived from the granulation raw material may be suitably determined, and is not limited as long as the effects of the present invention can be obtained.

In the present invention, from the viewpoint of improving the dispersibility of the polysaccharide thickener-containing preparation in a liquid, the proportions of pullulan, guar gum, guar gum decomposition product, gum arabic, gum ghatti, soybean polysaccharides, pectin, alginic acid and derivatives thereof, and the starch decomposition product having a DE of 1 or more and 50 or less derived from the binder liquid(s) in the polysaccharide thickener-containing preparation are such that the total amount of starch decomposition product having a DE of 1 or more and 50 or less is preferably 0.03 to 40 parts by mass, more preferably 0.05 to 35 parts by mass, further preferably 0.1 to 32 parts by mass, further more preferably 0.12 to 30 parts by mass, particularly preferably 0.15 to 28 parts by mass, and more particularly preferably 0.15 to 25 parts by mass, per part by mass of at least one member selected from the group consisting of pullulan, guar gum, guar gum decomposition product, gum arabic, gum ghatti, soybean polysaccharides, pectin, and alginic acid and derivatives thereof (in total). Moreover, the proportions of pullulan, guar gum, guar gum decomposition product, gum arabic, gum ghatti, soybean polysaccharides, pectin, alginic acid and derivatives thereof, and the starch decomposition product having a DE of 1 or more and 50 or less derived from the binder liquid(s) are such that the total amount of starch decomposition product having a DE of 1 or more and 50 or less may be, for example, 0.05 to 20 parts by mass, 0.1 to 15 parts by mass, 0.12 to 10 parts by mass, 0.15 to 8 parts by mass, 0.15 to 6 parts by mass, or the like, per part by mass of at least one member selected from the group consisting of pullulan, guar gum, guar gum decomposition product, gum arabic, gum ghatti, soybean polysaccharides, pectin, and alginic acid and derivatives thereof (in total). However, the present invention is not limited thereto.

The polysaccharide thickener-containing preparation produced in the present invention may contain a metal salt, an emulsifier, etc., in order to further improve dispersibility. The timing of adding a metal salt, an emulsifier, etc., is also not particularly limited. For example, the polysaccharide thickener-containing preparation may be produced by incorporating a metal salt, an emulsifier, and/or the like as a component of the granulation raw material when granulating a polysaccharide thickener; or mixing a metal salt, an emulsifier, and/or the like with a polysaccharide thickener after granulation.

The type of metal salt is not particularly limited. Examples include potassium salts, sodium salts, calcium salts, magnesium salts, and the like. Specific examples include chlorides, hydroxides, lactates, citrates, gluconates, phosphates, sulfates, and malates thereof, and the like. In the present invention, it is preferable to use a metal salt that is allowed to be used in a food or drink, or a pharmaceutical product for oral administration. Examples of such metal salts include calcium chloride, potassium chloride, sodium chloride, magnesium chloride, calcium lactate, sodium citrate, sodium gluconate, potassium gluconate, and the like. At least one member selected from the group consisting of calcium chloride, potassium chloride, sodium citrate, and sodium gluconate is a preferable metal salt. These metal salts may be used singly, or in a combination of two or more.

The type of emulsifier is also not particularly limited. Examples include sucrose fatty acid esters, glycerin fatty acid esters (e.g., monoglycerin fatty acid esters, polyglycerin fatty acid esters, polyglycerin condensed ricinoleic acid esters, and organic acid monoglycerides), propylene glycol fatty acid ester, sorbitan fatty acid ester, lecithin, enzymatically decomposed lecithin, enzymatically modified lecithin, sodium stearoyl lactylate, calcium stearoyl lactylate, quillaja extract, saponins, polysorbates (e.g., polysorbate 20, polysorbate 60, polysorbate 65, and polysorbate 80), and the like. These emulsifiers may be used singly, or in a combination of two or more.

The metal salt content and the emulsifier content in the polysaccharide thickener-containing preparation are not particularly limited. When the polysaccharide thickener-containing preparation contains a metal salt and/or an emulsifier, the metal salt content in the polysaccharide thickener-containing preparation is, for example, 0.5 to 10 mass%, preferably 0.8 to 8 mass%, more preferably 0.8 to 7 mass%, further preferably 1 to 6 mass%, particularly preferably 1 to 5.5 mass%, and more particularly preferably 1.5 to 5.5 mass%. The emulsifier content in the polysaccharide thickener-containing preparation is, for example, 0.1 to 10 mass%, and preferably 0.5 to 8 mass%. The polysaccharide thickener-containing preparation produced in the present invention may contain a nutritional component, a functional component, etc. Examples of nutritional components and functional components include vitamins, minerals, amino acids, peptides, proteins, collagens, and the like. The timing of adding these components is also not particularly limited. For example, the polysaccharide thickener-containing preparation may be produced by incorporating a nutritional component, a functional component, and/or the like as a component of the granulation raw material when granulating a polysaccharide thickener; or mixing a nutritional component, a functional component, and/or the like with a polysaccharide thickener after granulation.

An example of the present invention is described below. However, the present invention is not limited thereto.

For example, when the polysaccharide thickener-containing preparation of the present invention is produced by one-step granulation using a granulation raw material comprising a polysaccharide thickener, an excipient, and a metal salt, the polysaccharide thickener content in the granulation raw material is, for example, 5 to 90 mass%, preferably 10 to 85 mass%, more preferably 10 to 80 mass%, further preferably 15 to 80 mass%, further more preferably 18 to 80 mass%, particularly preferably 18 to 75 mass%, more particularly preferably 20 to 75 mass%, and even more particularly preferably 25 to 75 mass%. The phrase "the polysaccharide thickener content in the granulation raw material" as used herein is a value that does not contain dextrin. The excipient content in the granulation raw material is, for example, 9 to 90 mass% or 10 to 90 mass%, preferably 15 to 85 mass%, more preferably 18 to 80 mass%, further preferably 20 to 80 mass%, further more preferably 25 to 78 mass%, and particularly preferably 25 to 70 mass%. The metal salt content in the granulation raw materials is, for example, 0.5 to 10 mass%, preferably 0.8 to 8 mass%, more preferably 0.8 to 7 mass%, further preferably 1 to 6 mass%, and further more preferably 1 to 5.5 mass%.

As described above, the polysaccharide thickener-containing preparation of the present invention may be a granulated product itself obtained by granulating a granulation raw material as described above. Alternatively, the polysaccharide thickener-containing preparation may be obtained by, for example, mixing a granulated product obtained by granulating a granulation raw material with a metal salt etc., as necessary, as described above.

The polysaccharide thickener-containing preparation obtained by the production method of the present invention is excellent in dispersibility in a liquid, such as water. The preparation thus has an advantage such that it can be suitably used as a polysaccharide thickener-containing preparation used under conditions of not only rapid stirring with a machine, but also weak stirring, such as manually stirring (e.g., slow stirring conditions). For example, the polysaccharide thickener-containing preparation obtained by the production method of the present invention can be suitably used as a polysaccharide thickener-containing preparation used under conditions of stirring at a rotation speed of 400 rpm or less, or further conditions of stirring at 180 to 300 rpm. From this point of view, it can be said that the polysaccharide thickener-containing preparation of the present invention is, for example, preferably a polysaccharide thickener-containing preparation that can be used under conditions of stirring at a rotation speed of 400 rpm or less, or further conditions of stirring at 180 to 300 rpm when 2 g of the polysaccharide thickener-containing preparation is dispersed in 100 g of water (20°C).

Moreover, since the polysaccharide thickener-containing preparation obtained by the production method of the present invention has excellent dispersibility as described above, it has an advantage such that the inherent thickening function or gelling function of the polysaccharide thickener can be exhibited promptly by adding the preparation to a liquid, such as water. In particular, when a polysaccharide thickener that is soluble in water at a temperature around room temperature (e.g., 20°C), for example, at least one member selected from the group consisting of xanthan gum, carrageenan, guar gum, tara gum, welan gum, pectin, alginic acid and derivatives thereof, and starches, is used as a polysaccharide thickener, such a polysaccharide thickener generally tends to get lumpy; however, the production method of the present invention enables these polysaccharide thickeners to easily dissolve in a liquid, such as water, and thicken the liquid promptly.

The polysaccharide thickener-containing preparation obtained by the production method of the present invention can be widely used in various fields. For example, the preparation can be used in fields including the food and drink field, the pharmaceutical field (e.g., pharmaceutical products and quasi-drugs), the cosmetic field (e.g., makeup products, skin care products, perfumes, shampoos, conditioners, toothpaste, soap, and bath additives), the household product field (e.g., cleaning agents), the agricultural field (e.g., agricultural chemicals), the industrial field (e.g., paints), and the civil engineering field (e.g., cements, concrete, and soil purification agents). Among these fields, the food and drink field is preferable. The target foods and drinks are not particularly limited. Examples include water, tea drinks, soft drinks, fruit-juice-containing drinks, coffee drinks, milk drinks, nutritionally balanced drinks, isotonic drinks, functional drinks, vitamin supplement drinks, powdered drinks, alcoholic drinks, soup, miso soup, stew, curry, porridge, concentrated liquid food, enteral nutrition, pureed food, minced food, mousse food, paste food, seasonings, desserts, confectionery, frozen dessert, jam, processed meat food, processed farm food, processed fish-meat food, noodles, daily dishes, and like foods and drinks.

Further, the polysaccharide thickener-containing preparation produced by the production method of the present invention is preferably used for a liquid (desirably, a liquid food or drink) having a water content of 50 mass% or more, and more preferably a liquid (desirably, a liquid food or drink) having a water content of 60 mass% or more. This is because the polysaccharide thickener contained in the polysaccharide thickener-containing preparation can sufficiently swell and hydrate to exhibit a function (e.g., thickening function or gelling function) inherent to the polysaccharide thickener. When the water content of an object to be thickened or gelatinized is below the above value, a liquid, such as water, can be suitably added, and the polysaccharide thickener-containing preparation can then be used.

The form of the polysaccharide thickener-containing preparation obtained by the production method of the present invention is also not limited; and is, for example, preferably in a solid form, such as a powder, granules, or a tablet. The form of the preparation may be suitably determined according to the intended use. From the viewpoint of better usability, the polysaccharide thickener-containing preparation is, for example, preferably in a powder form or a granular form, and more preferably a granular form.

The polysaccharide thickener-containing preparation obtained by the production method of the present invention is also suitable as a thickening agent for increasing the viscosity of a target composition, because it can promptly exhibit the inherent thickening function of the polysaccharide thickener when added to a liquid, such as water. Further, since the preparation can promptly thicken a target composition even under conditions of weak stirring, such as manually stirring, it is useful as a thickening agent (e.g., a food thickener) for persons with reduced chewing function and/or reduced swallowing function to thicken food so that these persons can eat. Similarly, the polysaccharide thickener-containing preparation obtained by the production method of the present invention is also suitable as a gelling agent, because it can promptly exhibit the inherent gelling function of the polysaccharide thickener when added to a liquid, such as water.

The present invention also relates to a method for improving the dispersibility of a polysaccharide thickener-containing preparation in a liquid. This method can be performed by using a starch decomposition product having a DE of 1 or more and 50 or less, and at least one member selected from the group consisting of pullulan, guar gum, guar gum decomposition product, gum arabic, gum ghatti, soybean polysaccharides, pectin, and alginic acid and derivatives thereof as components of one or more binder liquids when granulating a polysaccharide thickener using binder liquid(s). More specifically, the present invention provides a method for improving the dispersibility of a polysaccharide thickener-containing preparation in a liquid, comprising the step of granulating a polysaccharide thickener using one or more binder liquids, wherein a starch decomposition product having a DE of 1 or more and 50 or less, and at least one member selected from the group consisting of pullulan, guar gum, guar gum decomposition product, gum arabic, gum ghatti, soybean polysaccharides, pectin, and alginic acid and derivatives thereof are used as components of the binder liquid(s). The method comprising granulating a polysaccharide thickener using the binder liquid(s) can be performed in the same manner as in the method described above. The components usable in this method, the content thereof, the procedure, etc., may be as described in the method explained above. The present invention enables an improvement in the dispersibility of a polysaccharide thickener-containing preparation in a liquid as described above, which makes it possible for the polysaccharide thickener to exhibit its inherent thickening function or gelling function more satisfactorily.

### Examples

The present invention is explained in detail below with reference to the Examples; however, the present invention is not limited to these Examples.

Tests in the Examples were performed by the following methods.

### Dispersibility test of polysaccharide thickener-containing preparation

(1) 100 g of ion exchange water (20°C) was placed in a 200 ml beaker. 2 g of a prepared polysaccharide thickener-containing preparation was added thereto, and allowed to stand for 3, 5, or 10 seconds.
(2) Thereafter, the mixture was stirred for 30 seconds using a spatula. The mixture in the beaker was stirred four times per second (manually stirred at 240 rpm).
(3) Appearance of lumps after stirring for 30 seconds was visually observed and evaluated according to the following criteria.

+++: A very large number (criterion: 30 or more) of large lumps (criterion: a diameter of 5 mm or more) and/or small lumps were found.
++: A small number (criterion: less than 10) of large lumps (criterion: a diameter of 5 mm or more) were found; or, there were no large lumps, but a large number (criterion: 20 or more) of small lumps (criterion: a diameter of less than 5 mm) were found.
+: Small lumps (criterion: a diameter of less than 5 mm) were found (criterion: 10 or more to less than 20).
±: A small number (criterion: 5 or more to less than 10) of small lumps (criterion: a diameter of less than 5 mm) were found.
-: A very small number (less than 5) of small lumps (criterion: a diameter of less than 5 mm) were found; or, no lumps were found.

### Viscosity-increasing property test of polysaccharide thickener-containing preparation

(1) 100 g of tap water (20°C), milk (20°C), or tea (bottled tea on the market, 20°C) was placed in a 200 ml beaker. While stirring the tap water, milk, or tea using a spatula, 2 g of a prepared polysaccharide thickener-containing preparation was added thereto, and stirred for 30 seconds. The mixture in the beaker was stirred four times per second (manually stirred at 240 rpm).
(2) The mixture after stirring was transferred to a screw bottle, and allowed to stand. Viscosity change over time was then measured. Based on one minute after the addition of the polysaccharide thickener-containing preparation to tap water or milk (0 min), viscosity change over time was determined by measuring the viscosity at 0, 3, 5, 10, 15, 30, and 60 minutes; or, based on one minute after the addition of the polysaccharide thickener-containing preparation to tea (0 min), viscosity change over time was determined by measuring the viscosity at 0, 3, 5, and 10 minutes. The viscosity of the thus-obtained mixture (20°C) of a polysaccharide thickener-containing preparation and tap water, milk, or tea was measured by using a Brookfield rotational viscometer (TVB-10, made by Toki Sangyo Co., Ltd.) at 12 rpm for one minute.

### Experiment Example 1: Preparation of polysaccharide thickener-containing preparations

Polysaccharide thickener-containing preparations having the formulations shown in Table 1 were prepared.

### Preparation of binder liquids

As shown in Table 1, in Examples 1-1 to 1-6, pullulan and a starch decomposition product having a DE of 1 or more to 50 or less were used as components of a binder liquid. Specifically, in Examples 1-1 to 1-6, pullulan and a starch decomposition product having a DE of 1 or more to 50 or less were added and dissolved in ion exchange water (20°C) to prepare binder liquids. In the preparation of the polysaccharide thickener-containing preparations of Examples 1-1 to 1-6 and Comparative Example 1, the following binder liquids were used.
Example 1-1: An aqueous solution containing 3 mass% of pullulan and 4 mass% of a starch decomposition product having a DE of 4.
Example 1-2: An aqueous solution containing 3 mass% of pullulan and 4 mass% of a starch decomposition product having a DE of 8.
Example 1-3: An aqueous solution containing 3 mass% of pullulan and 4 mass% of a starch decomposition product having a DE of 11.
Example 1-4: An aqueous solution containing 3 mass% of pullulan and 4 mass% of a starch decomposition product having a DE of 18.
Example 1-5: An aqueous solution containing 3 mass% of pullulan and 4 mass% of a starch decomposition product having a DE of 22.
Example 1-6: An aqueous solution containing 3 mass% of pullulan and 4 mass% of a starch decomposition product having a DE of 25.
Comparative Example 1: An aqueous solution containing 3 mass% of pullulan.

### Preparation of polysaccharide thickener-containing preparations

The polysaccharide thickener-containing preparations of Examples 1-1 to 1-6 were each prepared by fluidized bed granulation by spraying 150 g of a prepared binder liquid to 289.5 g of a granulation raw material (raw material for granulation, hereinafter also referred to as granulation mixture) containing 84 g of powdery xanthan gum and 205.5 g of dextrin.

In this Experiment Example, dextrin was used as an excipient. The same applies to all of the following Experiment Examples.

On the other hand, the polysaccharide thickener-containing preparation of Comparative Example 1 was prepared by fluidized bed granulation by spraying 150 g of the prepared binder liquid to 295.5 g of a granulation mixture containing 84 g of powdery xanthan gum and 211.5 g of dextrin.

**Table 1**

| Formulation of polysaccharide thickener-containing preparation | Example 1-1 | Example 1-2 | Example 1-3 | Example 1-4 | Example 1-5 | Example 1-6 | Comparative Example 1 |
|---|---|---|---|---|---|---|---|
| Mass% | | | | | | | |
| Xanthan gum | 28 | 28 | 28 | 28 | 28 | 28 | 28 |
| Pullulan | 1.5 | 1.5 | 1.5 | 1.5 | 1.5 | 1.5 | 1.5 |
| Starch decomposition product (DE4) | 2 | | | | | | |
| Starch decomposition product (DE8) | | 2 | | | | | |
| Starch decomposition product (DE11) | | | 2 | | | | |
| Starch decomposition product (DE18) | | | | 2 | | | |
| Starch decomposition product (DE22) | | | | | 2 | | |
| Starch decomposition product (DE25) | | | | | | 2 | |
| Dextrin | 68.5 | 68.5 | 68.5 | 68.5 | 68.5 | 68.5 | 70.5 |
| Total | 100 | 100 | 100 | 100 | 100 | 100 | 100 |

The prepared polysaccharide thickener-containing preparations were subjected to a dispersibility test and a viscosity-increasing property test. Table 2 shows the results of the dispersibility test, and Table 3 shows the results of the viscosity-increasing property test.

**Table 2**

| | Evaluation of dispersibility (Stirring after allowed to stand for three seconds) |
|---|---|
| Example 1-1 | - (Fig. 1) |
| Example 1-2 | - |
| Example 1-3 | - |
| Example 1-4 | - |
| Example 1-5 | - |
| Example 1-6 | - |
| Comparative Example 1 | ++ (Fig. 2) |

**Table 3**

| Tap water | Viscosity (mPa·s) | | | | | | |
|---|---|---|---|---|---|---|---|
| | 0 min. | 3 min. | 5 min. | 10 min. | 15 min. | 30 min. | 60 min. |
| Example 1-1 | 660 | 2090 | 2390 | 2880 | 3020 | 3110 | 3200 |
| Example 1-2 | 620 | 1960 | 2420 | 2890 | 3000 | 3040 | 3060 |
| Example 1-3 | 700 | 2080 | 2560 | 2890 | 2990 | 3000 | 3100 |
| Example 1-4 | 670 | 2030 | 2460 | 2870 | 2990 | 3140 | 3160 |
| Example 1-5 | 753 | 2050 | 2530 | 2980 | 3150 | 3250 | 3210 |
| Example 1-6 | 650 | 2010 | 2370 | 2890 | 3030 | 3060 | 3160 |

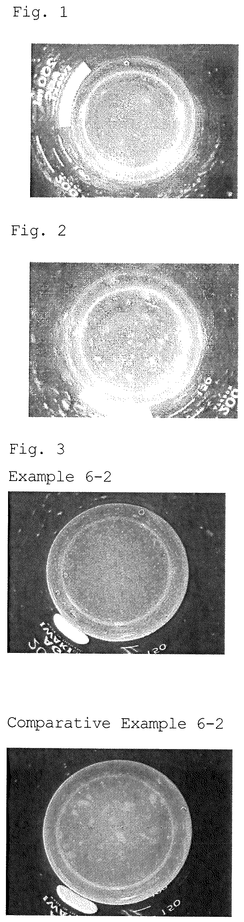

In the dispersibility test according to the present Examples, the polysaccharide thickener-containing preparation was allowed to stand for three seconds after addition to water, and then further stirred under weak stirring conditions, i.e., manually stirring. Thus, this is a severe test in which lumps are very easily generated. Accordingly, a large number (20 or more) of small lumps were generated in the polysaccharide thickener-containing preparation of Comparative Example 1 (Fig. 2).

The results revealed that when the polysaccharide thickener-containing preparation (Comparative Example 1) that had been granulated using the binder liquid containing pullulan alone was added to water, generation of lumps was not sufficiently suppressed.

In contrast, when each of the polysaccharide thickener-containing preparations (Examples 1-1 to 1-6) that had been granulated using a binder liquid containing pullulan and a starch decomposition product having a DE of 1 or more to 50 or less was added to water, generation of lumps was significantly suppressed. The results of the dispersibility test indicated that lumps were not generated; or, that even if lumps were generated, the size of the lumps was small, or the number of lumps was very low.

Specifically, when the polysaccharide thickener-containing preparation of Example 1-1, 1-2, or 1-5 was used, lumps were not formed (Fig. 1); and when the polysaccharide thickener-containing preparation of Example 1-3, 1-4, or 1-6 was used, one lump having a size of less than 2 mm was formed.

Additionally, as shown in Table 3, the polysaccharide thickener-containing preparations of Examples 1-1 to 1-6 had excellent viscosity-increasing properties. These Examples confirmed the production of polysaccharide thickener-containing preparations having improved dispersibility and being excellent in exhibiting functions (thickening function and gelling function) inherent to thickening polysaccharides (effectively exhibiting functions inherent to thickening polysaccharides).

### Experiment Example 2: Preparation of polysaccharide thickener-containing preparations

Polysaccharide thickener-containing preparations having the formulations shown in Table 4 were prepared.

### Preparation of binder liquids

As shown in Table 4, in Example 2, pullulan and a starch decomposition product having a DE of 22 were used as components of a binder liquid. Specifically, in Example 2, pullulan and a starch decomposition product having a DE of 22 were added and dissolved in ion exchange water (20°C) to prepare a binder liquid. In the preparation of the polysaccharide thickener-containing preparations of Example 2 and Comparative
Examples 2-1 to 2-3, the following binder liquids were used.
Example 2: An aqueous solution containing 4 mass% of pullulan and 4 mass% of a starch decomposition product having a DE of 22.
Comparative Example 2-1: An aqueous solution containing 4 mass% of pullulan.
Comparative Example 2-2: An aqueous solution containing 8 mass% of pullulan.
Comparative Example 2-3: An aqueous solution containing 8 mass% of a starch decomposition product having a DE of 22.

### Preparation of polysaccharide thickener-containing preparations

The polysaccharide thickener-containing preparations of Example 2 and Comparative Examples 2-2 to 2-3 were each prepared by fluidized bed granulation by spraying 150 g of a prepared binder liquid to 288 g of a granulation raw material (raw material for granulation, hereinafter also referred to as granulation mixture) containing 99 g of powdery xanthan gum and 189 g of dextrin.

On the other hand, the polysaccharide thickener-containing preparation of Comparative Example 2-1 was prepared by fluidized bed granulation by spraying 150 g of the prepared binder liquid to 294 g of a granulation mixture containing 99 g of powdery xanthan gum and 195 g of dextrin.

**Table 4**

| Formulation of polysaccharide thickener-containing preparation | Example 2 | Comparative Example 2-1 | Comparative Example 2-2 | Comparative Example 2-3 |
|---|---|---|---|---|
| Mass% | | | | |
| Xanthan gum | 33 | 33 | 33 | 33 |
| Pullulan | 2 | 2 | 4 | - |
| Starch decomposition product (DE22) | 2 | - | - | 4 |
| Dextrin | 63 | 65 | 63 | 63 |
| Total | 100 | 100 | 100 | 100 |

The prepared polysaccharide thickener-containing preparations were subjected to a dispersibility test and a viscosity-increasing property test. Table 5 shows the results of the dispersibility test, and Tables 6 and 7 show the results of the viscosity-increasing property test.

**Table 5**

| | Evaluation of dispersibility | |
|---|---|---|
| | Stirring after allowed to stand for three seconds | Stirring after allowed to stand for five seconds |
| Example 2 | - | - |
| Comparative Example 2-1 | + | ++ |
| Comparative Example 2-2 | - (Presence of non-swelled particle) | ± |
| Comparative Example 2-3 | ++ | +++ |

**Table 6**

| Tap water | Viscosity (mPa·s) | | | | | | |
|---|---|---|---|---|---|---|---|
| | 0 min. | 3 min. | 5 min. | 10 min. | 15 min. | 30 min. | 60 min. |
| Example 2 | 670 | 3070 | 3840 | 4130 | 4290 | 4440 | 4530 |
| Comparative Example 2-2 | 420 | 2540 | 3250 | 3960 | 4210 | 4290 | 4310 |

**Table 7**

| Milk | Viscosity (mPa·s) | | | | | | |
|---|---|---|---|---|---|---|---|
| | 0 min. | 3 min. | 5 min. | 10 min. | 15 min. | 30 min. | 60 min. |
| Example 2 | 26 | 272 | 603 | 1556 | 2280 | 3020 | 3860 |
| Comparative Example 2-2 | 0 | 118 | 271 | 650 | 1220 | 2010 | 2340 |

As shown in Table 5, when the mixture prepared using the polysaccharide thickener-containing preparation (Comparative Example 2-1) that had been produced by granulating a thickening polysaccharide (xanthan gum) using the binder liquid containing 4 mass% of pullulan alone was stirred after standing for three seconds after addition of the preparation, a large number (10 or more) of small lumps were generated; and when the mixture was stirred after standing for five seconds after addition, a large number (20 or more) of small lumps were generated. When the mixture prepared using the polysaccharide thickener-containing preparation (Comparative Example 2-2) that had been produced by granulating a thickening polysaccharide (xanthan gum) using the binder liquid containing 8 mass% of pullulan alone was stirred after standing for three seconds after addition of the preparation, no remarkable lumps were observed, but particles remained in a non-swelled state. When the mixture was stirred after standing for five seconds after addition, five small lumps were generated.

When the mixture prepared using the polysaccharide thickener-containing preparation (Comparative Example 2-3) that had been produced by granulating a thickening polysaccharide (xanthan gum) using the binder liquid containing 8 mass% of a starch decomposition product having a DE of 22 alone was stirred after standing for three seconds after addition of the preparation, a large number (20 or more) of small lumps were generated. When the mixture was stirred after standing for five seconds after addition, a very large number (30 or more) of large and small lumps were generated.

The above results revealed that generation of lumps when the preparations of Comparative Examples 2-1 to 2-3 were each added to water was not sufficiently suppressed.

In contrast, as shown in Table 5, when the mixture prepared using the polysaccharide thickener-containing preparation (Example 2) that had been produced by granulating a thickening polysaccharide (xanthan gum) using the binder liquid containing pullulan and a starch decomposition product having a DE of 22 was stirred after standing for three seconds after addition of the preparation, lumps were not generated. When the mixture was stirred after standing for five seconds after addition, lumps were generated; however, the size of each lump was less than 2 mm, and the number of lumps was only two.

The above results revealed that generation of lumps when the preparation of Example 2 was added to water was significantly suppressed.

In Comparative Example 2-2, although the number of generated lumps was relatively low, particles were present in a non-swelled state when the mixture was stirred after standing for three seconds after addition, as described above. As shown in Tables 6 and 7, Comparative Example 2-2 showed a poor viscosity-increasing property against tap water and milk; in particular, a significantly poor viscosity-increasing property against milk, as compared to Example 2. In contrast, Example 2 showed an excellent viscosity-increasing property against tap water and milk; in particular, a significantly excellent viscosity-increasing property against milk, as compared to Comparative Example 2-2.

### Experiment Example 3: Preparation of polysaccharide thickener-containing preparations

Polysaccharide thickener-containing preparations having the formulations shown in Table 8 were prepared.

### Preparation of binder liquids

As shown in Table 8, in Example 3, pullulan and a starch decomposition product having a DE of 30 were used as components of a binder liquid. Specifically, in Example 3, pullulan and a starch decomposition product having a DE of 30 were added and dissolved in ion exchange water (20°C) to prepare a binder liquid. In the preparation of the polysaccharide thickener-containing preparations of Example 3 and Comparative Example 3, the following binder liquids were used.
Example 3: An aqueous solution containing 4 mass% of pullulan and 4 mass% of a starch decomposition product having a DE of 30.
Comparative Example 3: An aqueous solution containing 4 mass% of pullulan and 4 mass% of a crystalline glucose hydrate having a DE of 100.

### Preparation of polysaccharide thickener-containing preparations

The polysaccharide thickener-containing preparations of Example 3 and Comparative Example 3 were each prepared by fluidized bed granulation by spraying 150 g of a prepared binder liquid to 288 g of a granulation raw material (raw material for granulation, hereinafter also referred to as granulation mixture) containing 99 g of a powdery xanthan gum and 189 g of dextrin.

**Table 8**

| Formulation of polysaccharide thickener-containing preparation | Example 3 | Comparative Example 3 |
|---|---|---|
| Mass% | | |
| Xanthan gum | 33 | 33 |
| Pullulan | 2 | 2 |
| Starch decomposition product (DE30) | 2 | - |
| Crystalline glucose hydrate (DE100) | - | 2 |
| Dextrin | 63 | 63 |
| Total | 100 | 100 |

The prepared polysaccharide thickener-containing preparations were subjected to a dispersibility test. Table 9 shows the results of the dispersibility test.

**Table 9**

| | Evaluation of dispersibility | |
|---|---|---|
| | Stirring after allowed to stand for three seconds | Stirring after allowed to stand for five seconds |
| Example 3 | - | - |
| Comparative Example 3 | + | ++ |

As shown in Table 9, when the mixture prepared using the polysaccharide thickener-containing preparation (Comparative Example 3) that had been produced by granulating a thickening polysaccharide (xanthan gum) using the binder liquid containing pullulan and a crystalline glucose hydrate having a DE of 100 was stirred after standing for three seconds after addition of the preparation, a large number (15 or more) of small lumps were generated; and when the mixture was stirred after standing for five seconds after addition, a large number (30 or more) of small lumps were generated.

The above results revealed that generation of lumps when the preparation of Comparative Example 3 was added to water was not sufficiently suppressed.

In contrast, as shown in Table 9, when the mixture prepared using the polysaccharide thickener-containing preparation (Example 3) that had been produced by granulating a thickening polysaccharide (xanthan gum) using the binder liquid containing pullulan and a starch decomposition product having a DE of 30 was stirred after standing for three seconds after addition of the preparation, lumps were not generated. When the mixture was stirred after standing for five seconds after addition, only one lump was generated, and the size of the lump was less than 2 mm.

The above results revealed that generation of lumps when the preparation of Example 3 was added to water was significantly suppressed.

### Experiment Example 4: Preparation of polysaccharide thickener-containing preparations

Polysaccharide thickener-containing preparations having the formulations shown in Table 10 were prepared.

### Preparation of binder liquids

As shown in Table 10, in Examples 4-1 to 4-3, pullulan and a starch decomposition product having a DE of 1 or more to 50 or less were used as components of a binder liquid. Specifically, in Examples 4-1 to 4-3, pullulan and a starch decomposition product having a DE of 1 or more to 50 or less were added and dissolved in ion exchange water (20°C) to prepare binder liquids. In the preparation of the polysaccharide thickener-containing preparations of Examples 4-1 to 4-3 and Comparative Example 4, the following binder liquids were used.
Example 4-1: An aqueous solution containing 4 mass% of pullulan and 4 mass% of a starch decomposition product having a DE of 22.
Example 4-2: An aqueous solution containing 4 mass% of pullulan and 6 mass% of a starch decomposition product having a DE of 22.
Example 4-3: An aqueous solution containing 4 mass% of pullulan and 6 mass% of a starch decomposition product having a DE of 2 to 5.
Comparative Example 4: An aqueous solution containing 4 mass% of pullulan.

### Preparation of polysaccharide thickener-containing preparations

The polysaccharide thickener-containing preparation of Example 4-1 was prepared by fluidized bed granulation by spraying 150 g of the prepared binder liquid to 288 g of a granulation raw material (raw material for granulation, hereinafter also referred to as granulation mixture) containing 60 g of powdery xanthan gum, 120 g of pregelatinized starch, and 108 g of dextrin.

The polysaccharide thickener-containing preparations of Examples 4-2 and 4-3 were each prepared by fluidized bed granulation by spraying 150 g of a prepared binder liquid to 285 g of a granulation mixture containing 60 g of powdery xanthan gum, 180 g of pregelatinized starch, and 45 g of dextrin.

On the other hand, the polysaccharide thickener-containing preparation of Comparative Example 4 was prepared by fluidized bed granulation by spraying 150 g of the prepared binder liquid to 294 g of a granulation mixture containing 60 g of powdery xanthan gum, 180 g of pregelatinized starch, and 54 g of dextrin.

**Table 10**

| Formulation of polysaccharide thickener-containing preparation | Example 4-1 | Example 4-2 | Example 4-3 | Comparative Example 4 |
|---|---|---|---|---|
| Mass% | | | | |
| Xanthan gum | 20 | 20 | 20 | 20 |
| Pregelatinized starch | 40 | 60 | 60 | 60 |
| Pullulan | 2 | 2 | 2 | 2 |
| Starch decomposition product (DE22) | 2 | 3 | - | - |
| Starch decomposition product (DE2 to 5) | - | - | 3 | - |
| Dextrin | 36 | 15 | 15 | 18 |
| Total | 100 | 100 | 100 | 100 |

The prepared polysaccharide thickener-containing preparations were subjected to a dispersibility test. Table 11 shows the results of the dispersibility test.

**Table 11**

| | Evaluation of dispersibility |
|---|---|
| | Stirring after allowed to stand for three seconds |
| Example 4-1 | - |
| Example 4-2 | - |
| Example 4-3 | - |
| Comparative Example 4 | + |

As shown in Table 11, when the mixture prepared using the polysaccharide thickener-containing preparation (Comparative Example 4) that had been produced by granulating thickening polysaccharides (xanthan gum and pregelatinized starch) using the binder liquid containing 4 mass% of pullulan alone was stirred after standing for three seconds after addition of the preparation, a large number (10 or more) of small lumps were generated.

The above results revealed that generation of lumps when the preparation of Comparative Example 4 was added to water was not sufficiently suppressed.

In contrast, as shown in Table 11, when the mixture prepared using the polysaccharide thickener-containing preparation (Examples 4-1 to 4-3) that had been produced by granulating thickening polysaccharides (xanthan gum and pregelatinized starch) using the binder liquid containing pullulan and a starch decomposition product having a DE of 1 or more to 50 or less was stirred after standing for three seconds after addition of the preparation, lumps were not generated.

The above results revealed that generation of lumps when the preparations of Examples 4-1 to 4-3 were each added to water was significantly suppressed. Thus, since generation of lumps was suppressed in Examples 4-1 to 4-3, it was confirmed that polysaccharide thickener-containing preparations being excellent in exhibiting functions (thickening function and gelling function) inherent to thickening polysaccharides (effectively exhibiting functions inherent to thickening polysaccharide) were formed.

### Experiment Example 5: Preparation of polysaccharide thickener-containing preparations

Polysaccharide thickener-containing preparations having the formulations shown in Table 12 were prepared.

### Preparations of binder liquids

As shown in Table 12, pullulan and a starch decomposition product having a DE of 8 were used as components of a binder liquid. Specifically, pullulan and a starch decomposition product having a DE of 8 were added and dissolved in ion exchange water (20°C) to prepare a binder liquid. In the preparation of the polysaccharide thickener-containing preparations of Examples 5-1 to 5-4, the following binder liquids were used.

Example 5-1: An aqueous solution containing 2 mass% of pullulan and 12 mass% of a starch decomposition product having a DE of 8. Example 5-2: An aqueous solution containing 4 mass% of pullulan and 8 mass% of a starch decomposition product having a DE of 8. Example 5-3: An aqueous solution containing 6 mass% of pullulan and 3 mass% of a starch decomposition product having a DE of 8. Example 5-4: An aqueous solution containing 8 mass% of pullulan and 1.6 mass% of a starch decomposition product having a DE of 8.

### Preparation of polysaccharide thickener-containing preparations

The polysaccharide thickener-containing preparation of Example 5-1 was prepared by fluidized bed granulation by spraying 150 g of the prepared binder liquid to 279 g of a granulation raw material (raw material for granulation, hereinafter also referred to as granulation mixture) containing 99 g of powdery xanthan gum and 180 g of dextrin.

The polysaccharide thickener-containing preparation of Example 5-2 was prepared by fluidized bed granulation by spraying 150 g of the prepared binder liquid to 282 g of a granulation raw material (raw material for granulation, hereinafter also referred to as granulation mixture) containing 108 g of powdery xanthan gum and 174 g of dextrin.

The polysaccharide thickener-containing preparation of Example 5-3 was prepared by fluidized bed granulation by spraying 150 g of the prepared binder liquid to 286.5 g of a granulation raw material (raw material for granulation, hereinafter also referred to as granulation mixture) containing 120 g of powdery xanthan gum and 166.5 g of dextrin.

The polysaccharide thickener-containing preparation of Example 5-4 was prepared by fluidized bed granulation by spraying 150 g of the prepared binder liquid to 285.6 g of a granulation raw material (raw material for granulation, hereinafter also referred to as granulation mixture) containing 120 g of powdery xanthan gum and 165.6 g of dextrin.

**Table 12**

| Formulation of polysaccharide thickener-containing preparation | Example 5-1 | Example 5-2 | Example 5-3 | Example 5-4 |
|---|---|---|---|---|
| Mass% | | | | |
| Xanthan gum | 33 | 36 | 40 | 40 |
| Pullulan | 1 | 2 | 3 | 4 |
| Starch decomposition product (DE8) | 6 | 4 | 1.5 | 0.8 |
| Dextrin | 60 | 58 | 55.5 | 55.2 |
| Total | 100 | 100 | 100 | 100 |

The prepared polysaccharide thickener-containing preparations were subjected to a dispersibility test. Table 13 shows the results of the dispersibility test.

**Table 13**

| | Evaluation of dispersibility |
|---|---|
| | Stirring after allowed to stand for three seconds |
| Example 5-1 | - |
| Example 5-2 | - |
| Example 5-3 | - |
| Example 5-4 | - |

As shown in Table 13, when the mixture prepared using the polysaccharide thickener-containing preparation (Examples 5-1 to 5-4) that had been produced by granulating a thickening polysaccharide (xanthan gum) using a binder liquid containing pullulan and a starch decomposition product having a DE of 8 was stirred after standing for three seconds after addition of the preparation, lumps were not generated.

The above results revealed that generation of lumps when the preparations of Examples 5-1 to 5-4 were each added to water was sufficiently suppressed.

### Experiment Example 6: Preparation of polysaccharide thickener-containing preparations

Polysaccharide thickener-containing preparations having the formulations shown in Table 14 were prepared.

### Preparation of binder liquids

As shown in Table 14, in Examples 6-1 and 6-2, pullulan and a starch decomposition product having a DE of 8 were used as components of a binder liquid. Specifically, in Examples 6-1 and 6-2, pullulan and a starch decomposition product having a DE of 8 were added and dissolved in ion exchange water (20°C) to prepare binder liquids. In the preparation of the polysaccharide thickener-containing preparations of Examples 6-1 to 6-2 and Comparative Examples 6-1 to 6-2, the following binder liquids were used.
Example 6-1: An aqueous solution containing 4 mass% of pullulan and 8 mass% of a starch decomposition product having a DE of 8.
Example 6-2: An aqueous solution containing 5 mass% of pullulan and 6 mass% of a starch decomposition product having a DE of 8.
Comparative Examples 6-1 and 6-2: Ion exchange water.

### Preparation of polysaccharide thickener-containing preparations

The polysaccharide thickener-containing preparation of Example 6-1 was prepared by fluidized bed granulation by spraying 150 g of the prepared binder liquid to 282 g of a granulation raw material (raw material for granulation, hereinafter also referred to as granulation mixture) containing 90 g of powdery welan gum and 192 g of dextrin.

The polysaccharide thickener-containing preparation of Example 6-2 was prepared by fluidized bed granulation by spraying 150 g of the prepared binder liquid to 283.5 g of a granulation raw material (raw material for granulation, hereinafter also referred to as granulation mixture) containing 90 g of powdery xanthan gum, 9 g of λ-carrageenan, and 184.5 g of dextrin.

On the other hand, the polysaccharide thickener-containing preparation of Comparative Example 6-1 was prepared by fluidized bed granulation by spraying 150 g of the ion exchange water as a binder liquid to 300 g of a granulation mixture containing 90 g of powdery welan gum and 210 g of dextrin.

The polysaccharide thickener-containing preparation of Comparative Example 6-2 was prepared by fluidized bed granulation by spraying 150 g of the ion exchange water as a binder liquid to 300 g of a granulation mixture containing 90 g of powdery xanthan gum, 9 g of λ-carrageenan, and 201 g of dextrin.

**Table 14**

| Formulation of polysaccharide thickener-containing | Example 6-1 | Example 6-2 | Comparative Example 6-1 | Comparative Example 6-2 |
|---|---|---|---|---|
| Mass% | | | | |
| Welan gum | 30 | - | 30 | - |
| Xanthan gum | - | 30 | - | 30 |
| λ-carrageenan | - | 3 | - | 3 |
| Pullulan | 2 | 2.5 | - | - |
| Starch decomposition product (DE8) | 4 | 3 | - | - |
| Dextrin | 64 | 61.5 | 70 | 67 |
| Total | 100 | 100 | 100 | 100 |

The prepared polysaccharide thickener-containing preparations were subjected to a dispersibility test. Tables 15 and 16 show the results of the dispersibility test.

**Table 15**

| | Evaluation of dispersibility |
|---|---|
| | Stirring after allowed to stand for 10 seconds |
| Example 6-1 | - |
| Comparative Example 6-1 | + |

**Table 16**

| | Evaluation of dispersibility |
|---|---|
| | Stirring after allowed to stand for three seconds |
| Example 6-2 | - (Fig. 3) |
| Comparative Example 6-2 | ++ (Fig. 3) |

As shown in Tables 15 and 16, when the mixture prepared using the polysaccharide thickener-containing preparation (Comparative Example 6-1) that had been produced by granulating a thickening polysaccharide (welan gum) using the ion exchange water as a binder liquid was stirred after standing for 10 seconds after addition the preparation, a large number (15 or more) of small lumps were generated. Additionally, when the mixture prepared using the polysaccharide thickener-containing preparation (Comparative Example 6-2) that had been produced by granulating thickening polysaccharides (xanthan gum and λ-carrageenan) using the ion exchange water as a binder liquid was stirred after standing for three seconds after addition of the preparation, a large number (25 or more, Fig. 3) of small lumps were generated.

The above results revealed that generation of lumps when the preparations of Comparative Examples 6-1 to 6-2 were each added to water was not sufficiently suppressed.

In contrast, as shown in Tables 15 and 16, when the mixture prepared using the polysaccharide thickener-containing preparation (Example 6-1) that had been produced by granulating a thickening polysaccharide (welan gum) using the binder liquid containing pullulan and a starch decomposition product having a DE of 8 was stirred after standing for 10 seconds after addition of the preparation, lumps were not generated. Additionally, when the mixture prepared using the polysaccharide thickener-containing preparation (Example 6-2) that had been produced by granulating thickening polysaccharides (xanthan gum and λ-carrageenan) using the binder liquid containing pullulan and a starch decomposition product having a DE of 8 was stirred after standing for three seconds after addition of the preparation, lumps were not generated (Fig. 3).

The above results revealed that generation of lumps when the preparations of Examples 6-1 to 6-2 were each added to water was significantly suppressed.

### Experiment Example 7: Preparation of polysaccharide thickener-containing preparations

Polysaccharide thickener-containing preparations having the formulations shown in Table 17 were prepared.

### Preparation of binder liquids

As shown in Table 17, in Examples 7-1 to 7-4, a starch decomposition product having a DE of 22, and pullulan, guar gum, gum arabic, or a soybean polysaccharide were used as components of a binder liquid. Specifically, in Examples 7-1 to 7-4, a starch decomposition product having a DE of 22, and pullulan, guar gum, gum arabic, or a soybean polysaccharide were added and dissolved in ion exchange water (20°C) to prepare binder liquids. In the preparation of the polysaccharide thickener-containing preparations of Examples 7-1 to 7-4 and Comparative Examples 7-1 to 7-2, the following binder liquids were used.
Example 7-1: An aqueous solution containing 5 mass% of pullulan and 5 mass% of a starch decomposition product having a DE of 22.
Example 7-2: An aqueous solution containing 0.2 mass% of guar gum and 5 mass% of a starch decomposition product having a DE of 22.
Example 7-3: An aqueous solution containing 5 mass% of gum arabic and 5 mass% of a starch decomposition product having a DE of 22.
Example 7-4: An aqueous solution containing 5 mass% of soybean polysaccharide and 5 mass% of a starch decomposition product having a DE of 22.
Comparative Examples 7-1: An aqueous solution containing 0.4 mass% of λ-carrageenan and 5 mass% of a starch decomposition product having a DE of 22.
Comparative Examples 7-2: An aqueous solution containing 5 mass% of a starch decomposition product having a DE of 22.

### Preparation of polysaccharide thickener-containing preparations

The polysaccharide thickener-containing preparations of Examples 7-1, 7-3, and 7-4 were each prepared by fluidized bed granulation by spraying 150 g of a prepared binder liquid to 285 g of a granulation raw material (raw material for granulation, hereinafter also referred to as granulation mixture) containing 90 g of powdery xanthan gum and 195 g of dextrin.

The polysaccharide thickener-containing preparation of Example 7-2 was prepared by fluidized bed granulation by spraying 150 g of the prepared binder liquid to 292.2 g of a granulation raw material (raw material for granulation, hereinafter also referred to as granulation mixture) containing 90 g of powdery xanthan gum and 202.2 g of dextrin.

On the other hand, the polysaccharide thickener-containing preparation of Comparative Example 7-1 was prepared by fluidized bed granulation by spraying 150 g of the prepared binder liquid to 291.9 g of a granulation mixture containing 90 g of powdery xanthan gum and 201.9 g of dextrin.

The polysaccharide thickener-containing preparation of Comparative Example 7-2 was prepared by fluidized bed granulation by spraying 150 g of the prepared binder liquid to 292.5 g of a granulation mixture containing 90 g of powdery xanthan gum and 202.5 g of dextrin.

**Table 17**

| Formulation of polysaccharide thickener-containing preparation | Example 7-1 | Example 7-2 | Example 7-3 | Example 7-4 | Comparative Example 7-1 | Comparative Example 7-2 |
|---|---|---|---|---|---|---|
| Mass% | | | | | | |
| Xanthan gum | 30 | 30 | 30 | 30 | 30 | 30 |
| Pullulan | 2.5 | - | - | - | - | - |
| Guar gum | - | 0.1 | - | - | - | - |
| Gum arabic | - | - | 2.5 | - | - | - |
| Soybean polysaccharide | - | - | - | 2.5 | - | - |
| λ-carrageenan | - | - | - | - | 0.2 | - |
| Starch decomposition product (DE22) | 2.5 | 2.5 | 2.5 | 2.5 | 2.5 | 2.5 |
| Dextrin | 65 | 67.4 | 65 | 65 | 67.3 | 67. 5 |
| Total | 100 | 100 | 100 | 100 | 100 | 100 |

The prepared polysaccharide thickener-containing preparations were subjected to a dispersibility test. Table 18 shows the results of the dispersibility test.

**Table 18**

| | Evaluation of dispersibility |
|---|---|
| | Stirring after allowed to stand for three seconds |
| Example 7-1 | - (Fig. 4) |
| Example 7-2 | - (Fig. 4) |
| Example 7-3 | - (Fig. 4) |
| Example 7-4 | - (Fig. 4) |
| Comparative Example 7-1 | + (Fig. 4) |
| Comparative Example 7-2 | ++ (Fig. 4) |

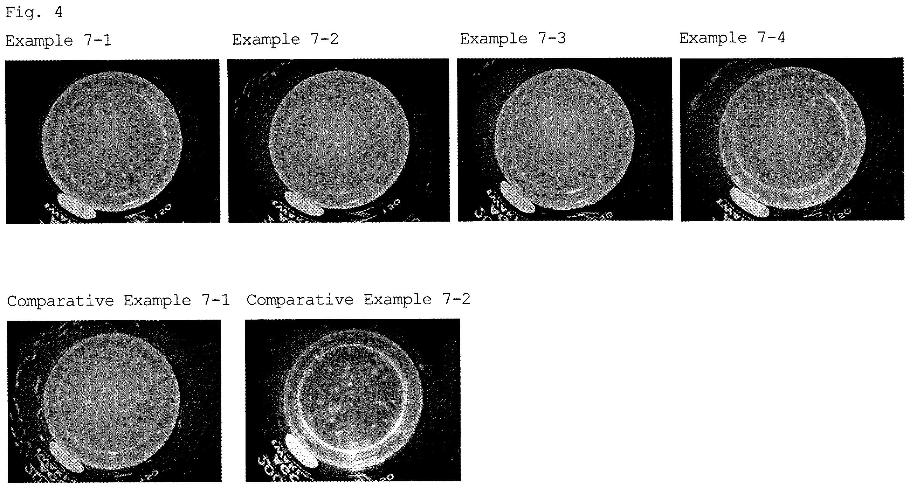

As shown in Table 18, when the mixture prepared using the polysaccharide thickener-containing preparation (Comparative Example 7-1) that had been produced by granulating a thickening polysaccharide (xanthan gum) using the binder liquid containing λ-carrageenan and a starch decomposition product having a DE of 22 was stirred after standing for three seconds after addition of the preparation, a large number (10 or more, Fig. 4) of small lumps were generated. Additionally, when the mixture prepared using the polysaccharide thickener-containing preparation (Comparative Example 7-2) that had been produced by granulating a thickening polysaccharide (xanthan gum) using the binder liquid containing a starch decomposition product having a DE of 22 alone was stirred after standing for three seconds after addition of the preparation, a large number (20 or more, Fig. 4) of small lumps were generated.

The above results revealed that generation of lumps when the preparations of Comparative Examples 7-1 to 7-2 were each added to water was not sufficiently suppressed.

In contrast, as shown in Table 18, when the mixture prepared using the polysaccharide thickener-containing preparation (Examples 7-1 to 7-4, Fig. 4) that had been produced by granulating a thickening polysaccharide (xanthan gum) using the binder liquid containing a starch decomposition product having a DE of 22 and pullulan, guar gum, gum arabic, or soybean polysaccharide was stirred after standing for three seconds after addition of the preparation, lumps were not generated.

The above results revealed that generation of lumps when the preparations of Examples 7-1 to 7-4 were each added to water was significantly suppressed.

### Experiment Example 8: Preparation of polysaccharide thickener-containing preparations

Polysaccharide thickener-containing preparations having the formulations shown in Table 19 were prepared.

### Preparation of binder liquids

As shown in Table 19, a starch decomposition product having a DE of 2 to 5 or 40, and guar gum, gum arabic, or soybean polysaccharide were used as components of a binder liquid. Specifically, a starch decomposition product having a DE of 2 to 5 or 40 and guar gum, gum arabic, or soybean polysaccharide were added and dissolved in ion exchange water (20°C) to prepare binder liquids. In the preparation of the polysaccharide thickener-containing preparations of Examples 8-1 to 8-6, the following binder liquids were used.
Example 8-1: An aqueous solution containing 0.2 mass% of guar gum and 4 mass% of a starch decomposition product having a DE of 2 to 5.
Example 8-2: An aqueous solution containing 0.2 mass% of guar gum and 4 mass% of a starch decomposition product having a DE of 40.
Example 8-3: An aqueous solution containing 5 mass% of gum arabic and 4 mass% of a starch decomposition product having a DE of 2 to 5.
Example 8-4: An aqueous solution containing 5 mass% of gum arabic and 4 mass% of a starch decomposition product having a DE of 40.
Example 8-5: An aqueous solution containing 5 mass% of soybean polysaccharide and 4 mass% of a starch decomposition product having a DE of 2 to 5.
Example 8-6: An aqueous solution containing 5 mass% of soybean polysaccharide and 4 mass% of a starch decomposition product having a DE of 40.

### Preparation of polysaccharide thickener-containing preparations

The polysaccharide thickener-containing preparations of Examples 8-1 to 8-2 were each prepared by fluidized bed granulation by spraying 150 g of a prepared binder liquid to 293.7 g of a granulation raw material (raw material for granulation, hereinafter also referred to as granulation mixture) containing 90 g of powdery xanthan gum and 203.7 g of dextrin.

The polysaccharide thickener-containing preparations of Examples 8-3 to 8-6 were each prepared by fluidized bed granulation by spraying 150 g of a prepared binder liquid to 286.5 g of a granulation raw material (raw material for granulation, hereinafter also referred to as granulation mixture) containing 90 g of powdery xanthan gum and 196.5 g of dextrin.

**Table 19**

| Formulation of polysaccharide thickener-containing preparation | Example 8-1 | Example 8-2 | Example 8-3 | Example 8-4 | Example 8-5 | Example 8-6 |
|---|---|---|---|---|---|---|
| Mass% | | | | | | |
| Xanthan gum | 30 | 30 | 30 | 30 | 30 | 30 |
| Guar gum | 0.1 | 0.1 | - | - | - | - |
| Gum arabic | - | - | 2.5 | 2.5 | - | - |
| Soybean polysaccharide | - | - | - | - | 2.5 | 2.5 |
| Starch decomposition product (DE2 to 5) | 2 | - | 2 | - | 2 | - |
| Starch decomposition product (DE40) | - | 2 | - | 2 | - | 2 |
| Dextrin | 67.9 | 67.9 | 65.5 | 65.5 | 65.5 | 65.5 |
| Total | 100 | 100 | 100 | 100 | 100 | 100 |

The prepared polysaccharide thickener-containing preparations were subjected to a dispersibility test. Table 20 shows the results of the dispersibility test.

**Table 20**

| | Evaluation of dispersibility |
|---|---|
| | Stirring after allowed to stand for three seconds |
| Example 8-1 | - (Fig. 5) |
| Example 8-2 | - (Fig. 5) |
| Example 8-3 | - (Fig. 5) |
| Example 8-4 | - (Fig. 5) |
| Example 8-5 | - (Fig. 5) |
| Example 8-6 | - (Fig. 5) |

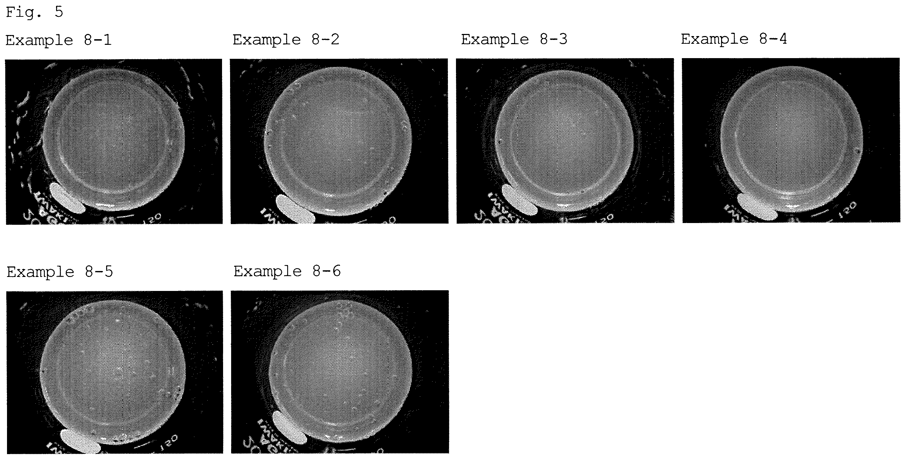

As shown in Table 20, when the mixture prepared using the polysaccharide thickener-containing preparation (Examples 8-1 to 8-6, Fig. 5) that had been produced by granulating a thickening polysaccharide (xanthan gum) using a binder liquid containing a starch decomposition product having a DE of 2 to 5 or 40, and guar gum, gum arabic, or soybean polysaccharide was stirred after standing for three seconds after addition of the preparation, lumps were not generated.

The above results revealed that generation of lumps when the preparations of Examples 8-1 to 8-6 were each added to water was significantly suppressed.

### Experiment Example 9: Preparation of polysaccharide thickener-containing preparations

Polysaccharide thickener-containing preparations having the formulations shown in Table 21 were prepared.

### Preparation of binder liquids

As shown in Table 21, a starch decomposition product having a DE of 22, and pullulan or gum arabic were used as components of a binder liquid. Specifically, a starch decomposition product having a DE of 22, and pullulan or gum arabic were added and dissolved in ion exchange water (20°C) to prepare binder liquids. In the preparation of the polysaccharide thickener-containing preparations of Examples 9-1 and 9-2, the following binder liquids were used.
Example 9-1: An aqueous solution containing 4 mass% of pullulan and 5 mass% of a starch decomposition product having a DE of 22.
Example 9-2: An aqueous solution containing 4 mass% of gum arabic and 5 mass% of a starch decomposition product having a DE of 22.

### Preparation of polysaccharide thickener-containing preparations

The polysaccharide thickener-containing preparations of Examples 9-1 and 9-2 were each prepared by fluidized bed granulation by spraying 150 g of a prepared binder liquid to 286.5 g of a granulation raw material (raw material for granulation, hereinafter also referred to as granulation mixture) containing 60 g of powdery xanthan gum and 226.5 g of dextrin.

**Table 21**

| Formulation of polysaccharide thickener-containing preparation | Example 9-1 | Example 9-2 |
|---|---|---|
| Mass% | | |
| Xanthan gum | 20 | 20 |
| Pullulan | 2 | - |
| Gum arabic | - | 2 |
| Starch decomposition product (DE22) | 2.5 | 2.5 |
| Dextrin | 75.5 | 75.5 |
| Total | 100 | 100 |

The prepared polysaccharide thickener-containing preparations were subjected to a dispersibility test and a viscosity-increasing property test. Table 22 shows the results of the dispersibility test, and Table 23 shows the results of the viscosity-increasing property test.

**Table 22**

| | Evaluation of dispersibility |
|---|---|
| | Stirring after allowed to stand for 10 seconds |
| Example 9-1 | - (Fig. 6) |
| Example 9-2 | - (Fig. 6) |

**Table 23**

| Tea | Viscosity (mPa·s) | | | |
|---|---|---|---|---|
| | 0 min. | 3 min. | 5 min. | 10 min. |
| Example 9-1 | 1200 | 3080 | 3170 | 3270 |
| Example 9-2 | 1270 | 3020 | 3260 | 3260 |

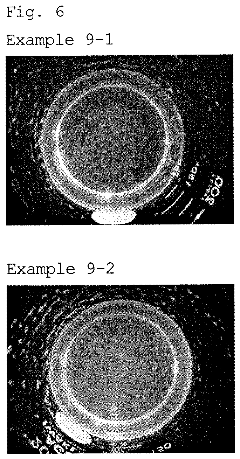

As shown in Table 22, even under extremely severe conditions such that the mixture prepared using the polysaccharide thickener-containing preparation (Example 9-1 or 9-2, Fig. 6) that had been produced by granulating a thickening polysaccharide (xanthan gum) using a binder liquid containing a starch decomposition product having a DE of 22 and pullulan or gum arabic was stirred after standing for 10 seconds after addition of the preparation, lumps were not generated; or, even if lumps were generated, the size of the lumps was small, or the number of lumps was 4. This revealed that generation of lumps when the preparation was added to water was significantly suppressed. Additionally, as shown in Table 23, the polysaccharide thickener-containing preparations of Examples 9-1 and 9-2 had excellent viscosity-increasing properties.

These Examples confirmed the production of polysaccharide thickener-containing preparations having improved dispersibility and being excellent in exhibiting functions (thickening function and gelling function) inherent to thickening polysaccharides (effectively exhibiting functions inherent to thickening polysaccharide).

## Claims

1. A method for producing a polysaccharide thickener-containing preparation, comprising the step of granulating a polysaccharide thickener using one or more binder liquids, wherein a starch decomposition product having a DE of 1 or more and 50 or less, and at least one member selected from the group consisting of pullulan, guar gum, guar gum decomposition product, gum arabic, gum ghatti, soybean polysaccharides, pectin, and alginic acid and derivatives thereof are used as components of the one or more binder liquids.

2. The method for producing a polysaccharide thickener-containing preparation according to claim 1, wherein a binder liquid comprising a starch decomposition product having a DE of 1 or more and 50 or less, and at least one member selected from the group consisting of pullulan, guar gum, guar gum decomposition product, gum arabic, gum ghatti, soybean polysaccharides, pectin, and alginic acid and derivatives thereof is used in the granulation step.

3. The method for producing a polysaccharide thickener-containing preparation according to claim 1 or 2, wherein the polysaccharide thickener comprises at least one member selected from the group consisting of xanthan gum, carrageenan, galactomannan, welan gum, pectin, gellan gum, alginic acid and derivatives thereof, and starches.

4. The method for producing a polysaccharide thickener-containing preparation according to any one of claims 1 to 3, wherein the proportion of the starch decomposition product having a DE of 1 or more and 50 or less relative to the at least one member selected from the group consisting of pullulan, guar gum, guar gum decomposition product, gum arabic, gum ghatti, soybean polysaccharides, pectin, and alginic acid and derivatives thereof, used as the components of the one or more binder liquids, is such that the amount of the starch decomposition product having a DE of 1 or more and 50 or less is 0.03 to 40 parts by mass per part by mass of the at least one member selected from the group consisting of pullulan, guar gum, guar gum decomposition product, gum arabic, gum ghatti, soybean polysaccharides, pectin, and alginic acid and derivatives thereof.

5. A method for improving the dispersibility of a polysaccharide thickener-containing preparation in a liquid, comprising the step of granulating a polysaccharide thickener using one or more binder liquids, wherein a starch decomposition product having a DE of 1 or more and 50 or less, and at least one member selected from the group consisting of pullulan, guar gum, guar gum decomposition product, gum arabic, gum ghatti, soybean polysaccharides, pectin, and alginic acid and derivatives thereof are used as components of the one or more binder liquids.
